# EUROPEAN PATENT APPLICATION

(11) **EP 1 792 630 A1**
(43) Date of publication of application: **06.06.2007**
(21) Application number: 05026307.8
(22) Date of filing: 02.12.2005
(51) Int. Cl.: A61K 47/48, A61K 31/455

(54) **Conjugates of amino acids and vitamin B3 for percutaneous delivery of vitamin B3**

(71) Applicant: THE PROCTER & GAMBLE COMPANY, Cincinnati, Ohio 45202 (US)
(72) Inventor: Ostacolo, Carmine, 83040 Guardia Lombardi (AV) (IT); Bernardi, Antonietta, 81025 Marcianise (CE) (IT); Laneri, Sonia, 80131 Napoli (IT); Sacchi, Antonia, 80123 Napoli (IT); Guarracino, Mario, 64029 Silvi Marina (TE) (IT); Santi, Patrizia, 43039 Salsomaggiore Terme (PR) (IT)
(74) Representative: Veronese, Pancrazio

(57) **Abstract**

The present invention relates to adducts of an amino acid or of a peptide with a vitamin B3 compound. Such adducts, thanks to the amino acid chain, easily penetrate the skin and are able to efficiently deliver the vitamin B3 compound to when applied topically. Such adducts can be used both in medical applications to treat vitamin B3 deficiency and in cosmetic application to exploit the skin care benefits of vitamin B3.

## Description

### FIELD OF THE INVENTION

The present invention relates to novel adducts of an amino acid or a peptide with a vitamin B3 compound selected from niacinamide and its derivatives. Such adducts, thanks to the amino acid chain, easily penetrate the skin trough the stratum corneum, up to the basal layer where they are hydrolyzed by the proteolitic enzymes of the skin thus releasing the vitamin B3 compound.

### BACKGROUND OF THE INVENTION

Many vitamin B3 compounds which can provide health and/or cosmetic benefits to the human and animal skin are often difficult to apply topically (i.e. on skin). In fact such compounds, in order to be effective, must penetrate up to the basal layer of the skin where the metabolic mechanisms of the cells take place. The human skin comprises several layers: the outermost is the stratum corneum, which comprises dead skin cells and makes up a substantial portion of the first protective barrier of the body. Most skin comprises a stratum corneum which is about 10-20 microns in thickness. However, some "durable" skin layers, such as heels or calluses, can comprise a stratum corneum which is from 100 to 150 microns thick. A topically applied composition, in order to be effective, must penetrate the stratum corneum and reach the lower layers of the skin. This is normally achieved when delivering pharmaceutical or cosmetic compositions via subcutaneous delivery, intramuscular delivery, as well as intravenous delivery. Less invasive procedures have now been developed and are widely utilized. Particularly friendly for the user is the topical application of creams or patches, which release the active components of the composition through the skin. A drawback of these patches is that certain active substances when topically applied to the skin, are not able to penetrate the stratum corneum and thus are absorbed at a very low rate, sometimes even too low to be effective.

Niacinamide (or nicotinamide or nicotinic acid amide) is a member of the vitamin B3 family of compounds, and is the physiologically active form of niacin (or nicotinic acid).

Niacin and niacinamide function in the body as components of two coenzymes: nicotinamide adenine dinucleotide (NAD) and nicotinamide adenine dinucleotide phosphate (NADP). Until recently, these vitamin B3 compounds were used exclusively to treat niacin deficiency and pellagra.

Today vitamin B3 compounds have also found use in the area of skin care actives. GB 1,370,236 describes compositions for skin lightening containing 0.5% to 10% niacin. U.S. Pat. No. 4,096,240 discloses the use of 0.1% to 10% niacinamide for skin lightening. WO 97/39733 describes Vitamin B3 compounds to be used in regulating the texture of human skin.

However, when topically applied to the skin, the penetration is very slow, in fact only about 2-4% of the applied vitamin B3 compound actually penetrates into the skin. Thus, there exists a need for cosmetic compositions comprising vitamin B3 compounds which provide improved skin penetration of said vitamin B3 compounds.

In the art there are several attempts to solve this problem. For example in case US 6,528,071 from The Procter & Gamble Company it is suggested to incorporate vitamin B3 compounds in a polar solvent so that the vitamin B3 compound exceeds the saturation solubility of the polar solvent.

This solution, although effective, can still be improved in fact polar solvents can pose stability problems when formulated into cosmetic compositions made primarily of lipophilic materials (i. e., forming a lipophilic continuous phase). When formulating such compositions, the polar solvents (e. g., polyhydric alcohols, water) necessary to dissolve the vitamin B3 compounds tend to separate from the lipophilic materials, causing the formation of messy looking bulk layers. Moreover, when formulating lipophilic stick compositions (e. g., lipsticks), such phase separation manifests itself as liquid beads along the surface of the stick composition. This can negatively affect consumer acceptability.

A solution to this has been presented in EP 1,152,742 from The Procter & Gamble Company where it is described how the stability of cosmetic compositions comprising a vitamin B3 compound, a polar solvent and a lipophilic continuous phase can be improved by incorporating a surfactant or surfactant mixtures which has a Krafft point at or below about 20°C and which form association structures such that the association structures thermodynamically bind the moisturizer/polar solvent and homogeneously absorb in the lipophilic matrix.

Although these solutions provide an improved absorption rate of the vitamin B3 compound, there is still room for further improvement, for example providing new materials which can be absorbed at an even faster rate (thus allowing the use of significantly lower amounts of the vitamin B3 compound in the compositions to be applied) and which can be easily formulated in simpler compositions not requiring complex solvent-surfactant systems in order to be physically stable.

### SUMMARY OF THE INVENTION

The present invention relates to novel adducts of an amino acid or a peptide with a vitamin B3 compound, selected from niacinamide and its derivatives, said adducts being formed via an imide bond.
The new adducts have the following general formula:

R₁-NX-R₂

wherein R₁ is the acylic radical of niacinamide or of a derivative thereof and R₂ is the acylic radical of an amino acid or of a peptide. X can be any radical and is preferably selected from -OH, -Alg (where Alg is any alogen atom), -CH2COOH, -CH2COOMe wherein Me is an alkali metal, and -H (hydrogen). Most preferably X is -H.

In one aspect the present invention relates to said adducts for use as a medicament.

In another aspect the present invention relates to a method for preparing said adducts.

In another aspect the present invention relates to the use of said adducts to prepare a composition to treat diseases related to vitamin B3 deficiency.

In another aspect the present invention relates to a cosmetic composition comprising said adducts.

### DETAILED DESCRIPTION OF THE INVENTION

### Vitamin B3 compound

The vitamin B3 compound to be used in the present invention is selected from niacinamide and its derivatives. Niacinamide has the following chemical structure:

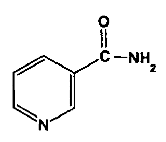

Exemplary niacinamide derivatives which can be used as vitamin B3 compounds in the present invention include ring substituted niacinamide, niacinamide N-oxides, salts of niacinamide.

Non-limiting examples of niacinamide derivatives useful herein are 2-chloronicotinamide, 6-aminonicotinamide, 6-methylnicotinamide, n-methyl-nicotinamide, n,n-diethylnicotinamide, n-(hydroxymethyl)-nicotinamide, quinolinic acid imide, nicotinanilide, n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Examples of the above niacinamide derivatives are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

Although not preferred, derivatives of niacinamide resulting from substitution of one of the amide group hydrogen atoms can be used herein, as for example e.g nicotinuric acid: 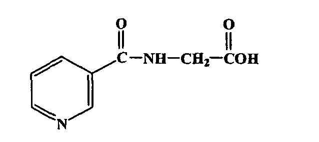 and nicotinyl hydroxamic acid:

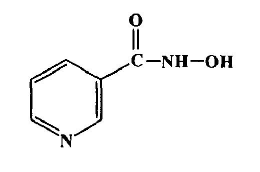

Salts of niacinamide and of any of the mentioned derivatives can also be used herein. As known to the skilled man, niacinamide can form either cations, when protonating a nitrogen atom, or anions when losing a hydrogen atom from the amidic nitrogen. Therefore niacinamide can form both e.g. alkali metal salts and salts with organic or inorganic anions. This applies of course also to any of the mentioned derivatives of niacinamide, and their salts can be used as well herein as vitamin B₃ compounds. Non limiting examples of salts of the vitamin B₃ compound useful herein include organic or inorganic salts, such as inorganic salts with anionic inorganic species (e.g., chloride, bromide, iodide, carbonate, preferably chloride), and organic carboxylic acid salts (including mono-, di- and tri- C1 - C18 carboxylic acid salts, e.g., acetate, salicylate, glycolate, lactate, malate, citrate, preferably monocarboxylic acid salts such as acetate). These and other salts of the vitamin B₃ compound can be readily prepared by the skilled artisan, for example, as described by W. Wenner, "The Reaction of L-Ascorbic and D-Isoascorbic Acid with Nicotinic Acid and Its Amide", J. Organic Chemistry, VOL. 14, 22-26 (1949). Wenner describes the synthesis of the ascorbic acid salt of niacinamide.

In a preferred embodiment, the ring nitrogen of the vitamin B3 compound is substantially chemically free (e.g., unbound and/or unhindered). More preferably, the vitamin B₃ compound is essentially uncomplexed. Therefore, if the vitamin B3 compound is in a salt or otherwise complexed form, such complex is preferably substantially reversible, more preferably essentially reversible, upon delivery of the composition to the skin. For example, such complex should be substantially reversible at a pH of from about 5.0 to about 6.0. Such reversibility can be readily determined by one having ordinary skill in the art.

Most preferably the vitamin B3 compound to be used in the present invention is niacinamide.

### Amino Acid or peptide

The term amino acid, as used in the present application, encompasses amino acids and their salts. Any amino acid can be used in the present invention. Amino acids are organic compounds comprising a carboxylic group and an amino group. Preferred amino acids are α-amino acids i.e. those amino acids wherein the amino group and the carboxylic group are linked to the same carbon atom. Particularly preferred amino acids are natural amino acids, i.e. the 20 amino acids which are well known as the building blocks of natural proteins. A list of these 20 amino acids is reported in table 1.

As apparent to the skilled man, said amino acids can be used also in their salt form or in their pyro (i.e. dehydrated) form.

All naturally occurring amino acids have the common general formula: 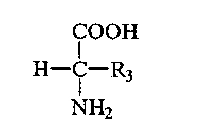 wherein R₃ is different for each member of the family, with the exception of proline which is the only one where the side chain forms a ring with the amino group:

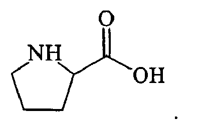

**Table 1 the 20 natural amino acids.**

| **Amino acid** | **Abbrev.** | **R₃ Side chain** |
|---|---|---|
| Alanine | Ala, A | -CH₃ |
| Cysteine | Cys, C | -CH₂SH |
| Aspartate | Asp, D | -CH₂COOH |
| Glutamate, glutamic acid | Glu, E | -CH₂CH₂COOH |
| Phenylalanine | Phe, F | -CH₂C₆H₅ |
| Glycine | Gly, G | -H |
| Histidine | His, H | -CH₂-C₃H₃N₂ |
| Isoleucine | Ile, I | -CH(CH₃)CH₂CH₃ |
| Lysine | Lys, K | -(CH₂)₄NH₂ |
| Leucine | Leu, L | -CH₂CH(CH₃)₂ |
| Methionine | Met, M | -CH₂CH₂SCH₃ |
| Asparagine | Asn, N | -CH₂CONH₂ |
| Proline | Pro, P | -CH₂CH₂CH₂-cycle see figure above. |
| Glutamine | Gln, Q | -CH₂CH₂CONH₂ |
| Arginine | Arg, R | -(CH₂)₃NH-C(NH)NH₂ |
| Serine | Ser, S | -CH₂OH |
| Threonine | Thr, T | -CH(OH)CH₃ |
| Valine | Val, V | -CH(CH₃)₂ |
| Tryptophan | Trp, W | -CH₂C₈H₅N |
| Tyrosine | Tyr, Y | -CH₂-C₆H₄OH |

Most preferred amino acids for use in the present invention are proline, glutamic acid and phenylalanine because they offer the best compatibility with the human skin and consequently the best penetration of the vitamin B3 compound.

As an alternative to amino acids, peptides can be used. Peptides are molecules still formed by two or more amino acids as described above chained together by a peptide bond and having a free carboxylic group at the end of the chain. Peptides are preferably formed by different units of the same amino acid and in general are preferably formed by 2 or 3 molecules of amino acid.

### The adduct

In one aspect the present invention relates to novel adducts between a vitamin B3 compound selected from niacinamide or a derivative thereof and an amino acid or a peptide. The adducts have the following general formula:

R₁-NX-R₂

wherein R₁ is an acylic radical of niacinamide or of a derivative thereof and R₂ is the acylic radical of an amino acid or of a peptide. X can be any radical and is preferably selected from -OH, -Alg (where Alg is any alogen atom), -CH₂COOH, -CH₂COOMe wherein Me is an alkali metal, and -H (hydrogen). Most preferably X is -H.

The schematic chemical reaction which forms the novel adducts can be seen as the condensation between 1 molecule of vitamin B3 compound and 1 molecule of amino acid or of peptide with the elimination of 1 molecule of water:

R₁-NHX + HO-R₂ → R₁-NX-R₂ + H₂O

In the following paragraphs it will be illustrated in detail how the novel adducts of the present invention can be obtained. In all cases niacinamide will be used as exemplary and preferred vitamin B3 compound. It is intended that the same processes can be applied to a niacinamide derivative as described above and that the use of a derivative will result in obtaining the respective adduct where the acylic radical R1 of the derivative will take the place of the acylic radical of the niacinamide.

The following examples will show in detail how adducts according to the present invention can be obtained from niacinamide and some exemplary amino acid. The skilled man, following these indications is of course able through routinary procedures to determine the optimal reaction conditions also for all other amino acids and peptides.

In general the adducts of the present invention can be obtained starting from the alkali metal salt (preferably a sodium or potassium salt) of niacinamide or of its derivative (obtainable via routinary techniques) and a reversibly protected form of the amino acid or peptide wherein the nitrogen of the amino group is sterically hindered. The reversible protection of the amino group is a routinary technique in the chemistry of amino acids and is usually done either by introducing a tert-butoxycarbonyl radical on the nitrogen atom of the amino group or, for dicarboxylic amino acids, by forming the dehydrated or "pyro" forms which will hydrolyze back to the hydrated form upon contact with water. Other protective radicals which are commonly introduced to sterically hinder the nitrogen atom of the amino group with techniques which are available to those skilled in the art are FMOC, acetyl, trifluoroacetyl, benzoyl, benzyloxycarbonyl, isobutyl, palmitoyl, trityl, 4,4-Dimethyl-2,6-dioxocyclohex-1-ylidene.

In case the amino acid or peptide comprises a functionalized side chain, a protective radical may be introduced also on said side chain. The choice of possible radicals to be used in this case is very broad and depends on the specific amino acid or peptide. The skilled man can use routinary techniques in order to select the appropriate protective radical and to introduce it in the molecule. Some exemplary protective radicals which can be used for protecting amino acids or peptides which comprise a functionalized side chain are tert-butoxycarbonyl, FMOC, 4-methoxy-2,3,6-trimethylbenzenesulfonyl, nitro, 2,2,4,6,7-pentamethyldihydrobenzofuran-5-sulfonyl, 2,2,5,7,8-pentamethylchroman-6-sulfonyl, tosyl, pentafluorophenyl, 3,4,6-tri-o-acetyl-2-(acetylamino)-deoxy-2-beta-glucopyranosyl, 2,4,6-trimethoxybenzyl, allyl, allyloxycarbonyl, adamantly, benzyl, 2,6-dichlorobenzyl, tert-butyl (t-Bu), 1-(4,4-dimethyl-2,6-dioxocyclohexylidene)-3-methylbutylaminobenzyl, 2-Phenylisopropyl, acetamidomethyl (Acm), tert-butylthio, methoxybenzyl, methylbenzyl, methoxytrityl, trityl, 2-Chloro-trityl, 1-(4,4-dimethyl-2,6-dioxocyclohex-1-ylidene)-3-methylbutyl, 1-(4,4-dimethyl-2,6-dioxocyclohex-1-ylidene)-ethyl, methyltrityl, N-Biotinyl-3-(2-(2-(3-aminopropyloxy)-etoxy)-etoxy)-propyl), 2-trymethylsilylethyl, 4,4'-dimethoxybenzhydryl, 2-Fluorenylmethoxycarbonyloxy-4-metoxybenzyl, acetyl, biotinyl, biotinyl-e-caproyl, benzyloxycarbonyl, 2-chlorobenzyloxycarbonyl, 2-bromobenzyloxycarbonyl, dinitrophenyl, methoxycoumarin, trifluoroacetyl, Phospho, O-Benzylphospho, bis-dimethylamino-oxo-phosphino, xantyl, cyclohexyl, sulfone, formyl, mesitylene-2-sulfonyl, 2,6-dichlorobenzyl, 2,6-dibromobenzyl.

In order to improve reaction yield the protected amino acid or peptide is preferably activated (by using techniques which are common in the chemistry of amino acids e.g. by forming an acylic chloride or by reacting it with carbonyildiimidazole) and then is reacted with the alkali metal salt of the niacinamide or its derivative in an appropriate solvent (preferably THF) thus forming the adduct of the present invention. The adduct is then purified using appropriate techniques which are available to the skilled man. In most cases the solvent is evaporated and the residue is recovered with a polar solvent (e.g. ethyl acetate), an alkaline water solution is added and the adduct is extracted twice from the solution with an immiscible organic solvent. The organic phase is dehydrated (e.g. on Na₂SO₄) filtered and finally the purified adduct is obtained after evaporation of the organic solvent. Optionally the resulting material can be further purified by chromatography as known in the art. The yield obtained is about 40 to 100%.

Starting materials are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

### Example 1 - Niacinamide-glutamic acid adduct

With reference to Scheme 1, starting material was L-Pyroglutamic Acid (3). This can be prepared from glutamic acid using routinary techniques or purchased from chemical suppliers as those mentioned above. A stirred dispersion of L-pyroglutamic acid (3) (2.17 g, 16.38 mmol) and saccharin (0.017 g) in hexamethyldisilazane (HMDS 1.32 g, 8.19 mmol) was refluxed for 2.5h. The residual amount of HMDS was then evaporated. The silyl ester obtained was dissolved in methylene dichloride, and oxalyl chloride (2.06g, 16.38 mmol) was added slowly in 30 minutes. The mixture was stirred at room Temperature for 60 minutes and refluxed for few minutes (until the end of gas evolution). The solvent and trimethylsilyl chloride were evaporated in vacuo giving intermediate 4 as a yellow oil in quantitative yield.

The other starting material was Niacinamide sodium salt (intermediate 2) which is prepared in the following way: to a suspension of 0,49 g of NaH (80% in mineral oil dispersion, 16.38 mmol) in 7 ml of THF dry, were slowly added, at 0°C, 2.0 g (16,38 mmol) of Niacinamide (1). The solution was stirred for 20 minutes. The product obtained was not isolated from the solution and was immediately reacted with the other intermediate 4 as follows.

Intermediate 4 (2.30 g, 16.38 mmol) was dissolved in 2 ml of THF dry and then added of solution containing intermediate 2. The solution was stirred for 1.5h. Then the solvent was evaporated in vacuum, the residue dissolved in Ethyl Acetate and added with aqueous solution of NaOH (0,5 N). The organic phase was extracted twice, then dried on Na2SO4, filtered and evaporated in vacuum. The crude product was purified by chromatography, using Ethyl acetate/Methanol 9/1 as eluent. 2.29 g (yield 60%) of a white solid (5) were obtained.

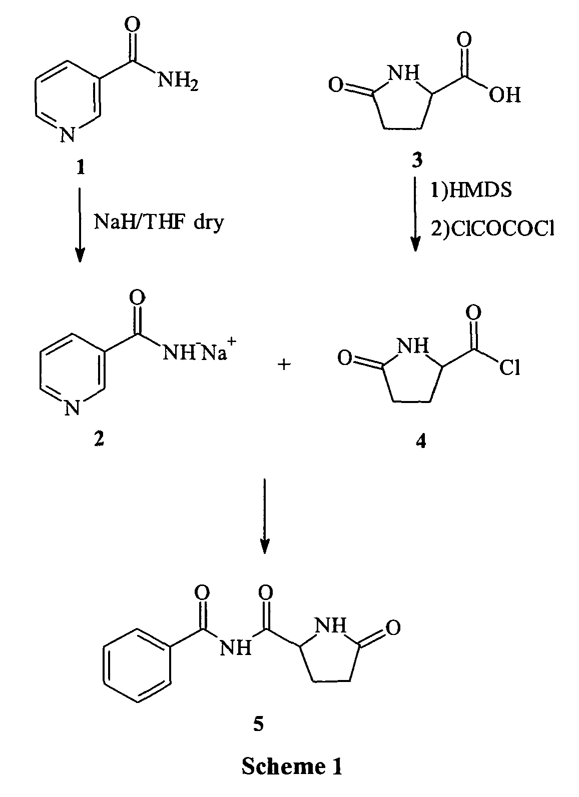

### Example 2 - Niacinamide-phenyl Alanine adduct

With reference to Scheme 2, starting material was L-Boc-Phenyl Alanine (6), a phenyl Alanine protected with a tert-butoxycarbonyl radical on the amino group which can be prepared with routinary techniques or purchased from mentioned chemicals suppliers. A solution of L-Boc-Phenyl alanine (4,34 g, 16,38 mmol) (6) in 10 ml of THF dry was slowly added of 2.92 g (18.02 mmol) of carbonyl diimidazole. The solution was stirred until effervescence disappeared. Then was added of the solution containing the intermediate 2 (niacinamide sodium salt prepared as in Example 1). The reaction was stirred for 3h at room temperature, then the solvent was evaporated and the residue was dissolved in Ethyl acetate and extracted twice with an aqueous solution of NaOH (0,5 N). The organic phases were collected, dried on Na₂SO₄, filtered and evaporated in vacuum. The crude product was purified in column chromatography using 9.5/0.5 Diethyl ether/Methanol as eluent. 3.30g (yield 55%) of 8 as a colorless oil were obtained.

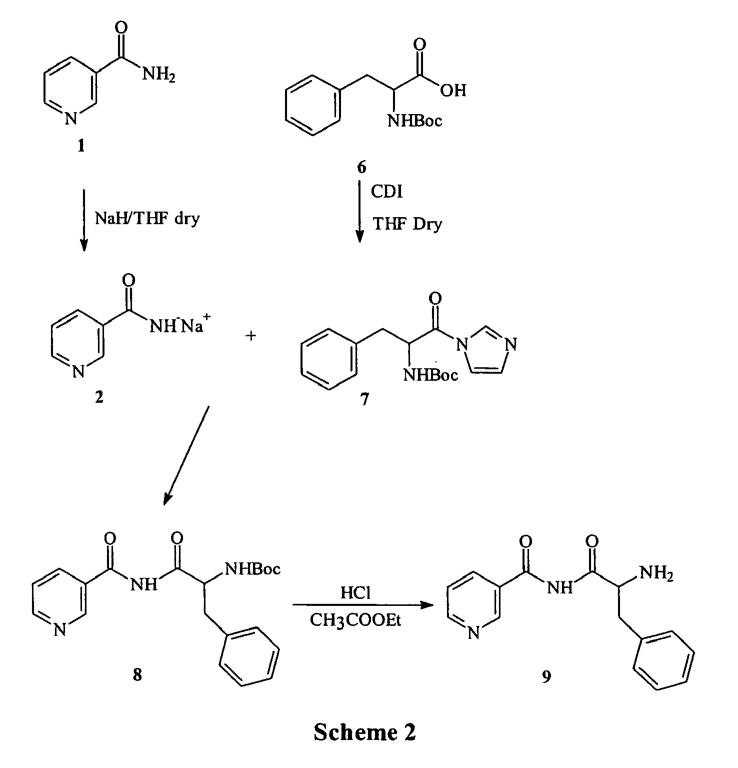

1.0 g (2.72 mmol) of 8 were dissolved in 20 ml of dry Ethyl acetate. 1.66ml of HCl (37% in aqueous solution) were added and the solution was stirred at room temperature for 45 minutes. The precipitate was collected by filtration and dried under reduced pressure for 1h. Then was dissolved in a aqueous solution saturated with Na₂HCO₃, and extracted three times with Ethyl acetate. The organic phases were collected, dried on Na₂SO₄, filtered and evaporated in vacuum. 0.73 g (yield 100%) of 9 were obtained as a white semisolid.

### Example 3 - Niacinamide - proline adduct

With reference to Scheme 3, starting material was L-Boc-proline (10), a protected proline with a tert- butoxycarbonyl radical on the amino group which can be prepared with routinary techniques or purchased from mentioned chemicals suppliers.

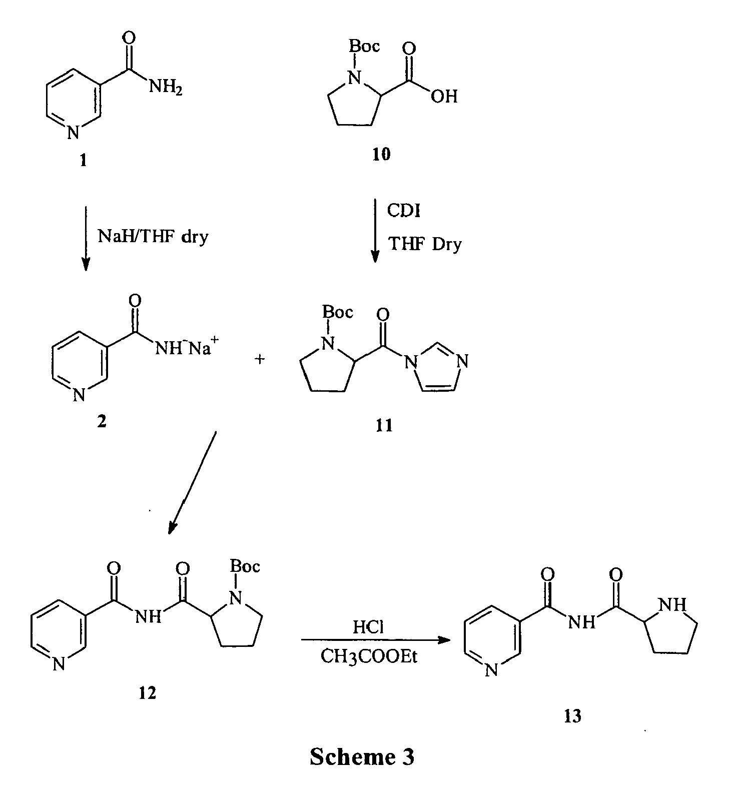

Intermediate (12) was obtained in the same manner intermediate 8 is obtained in example 2, by reacting 3.53g (16.38 mmol) of L-Boc-proline with 2.92g (18.02 mmol) of CDI in 10 ml of THF dry. The crude product was purified by column chromatography using 9.5/0.5 Diethyl ether/methanol as eluent. 3.12g (yield 58%) of 12 were obtained as a colorless oil. The adduct (13) was obtained using the same method used in example 2 starting from 1.0 g (3.13 mmol) of 12. 0.68g of adduct (yield 100%) were obtained as a white semisolid.

Surprisingly adducts according to the present invention effectively penetrate the skin and get hydrolyzed in the basal layer thus allowing effective topical delivery of the vitamin B3 compound into the body.

Without being bound to theory it is believed that the vitamin B3 compounds, per se, are too polar to efficiently penetrate the skin. The lipophilic character of the stratum corneum provides a significant barrier for the penetration of the vitamin B3 compounds. Their adducts with amino acids or peptides according to the present invention are much more lipophilic and therefore are able to penetrate the skin very effectively. Once the adduct reaches the basal layer, where the metabolic mechanisms of the cells take place, the vitamin B3 compound is released since the imide bond in vicinal position with the amino group of the amino acid or peptide is very similar to the peptidic bond of the proteins and can be hydrolyzed by the proteolitic enzymes of the skin.

Adducts according to the present invention are also easy to formulate in simple cosmetic bases and do not require specific complex solvent-surfactant system in order to be stabilized.

The adducts so obtained find particularly useful application in all those compositions which are used to deliver vitamin B3 to the human or animal body via topical application.

Compositions comprising an adduct according to the present invention can be used in the medical field to cure vitamin B3 deficiency and illnesses connected with it like pellagra.

Compositions comprising an adduct according to the present invention can also be used in the cosmetic field in order to provide skin care benefits. Cosmetic compositions comprising an adduct according to the present invention will typically comprise from 0.1% to 30%, preferably from 0.5% to 10%, more preferably from 1% to 5% by weight of the total composition of an adduct according to the present invention, along with common ingredients for cosmetic applications. In general, in order to provide the same efficacy in delivering a vitamin B3 compound to the body, cosmetic compositions according to the present invention will comprise the adducts at a lower concentration than the concentration of vitamin B3 compounds in prior art compositions.

An exemplary lipstick composition according to the present invention, can be prepared as follows (all percentages are weight percentages of the whole formulation):

| | |
|---|---|
| Polyisobutene | 30% |
| Isopropyl Myristate | 10% |
| Lanolin Oil | 53.5% |
| Candelilla Wax | 2.5% |
| Vitamin B3 adduct | 3% |
| Preservatives/pigments/perfumes/pH control agents. | 1% |

the ingredients are mixed at about 70°C and homogenized. The mixture is then cooled down.

An exemplary skin lightening cream according to the present invention, can be prepared as follows (all percentages are weight percentages of the whole formulation):

| | |
|---|---|
| Stearyl Alcohol | 1% |
| Glyceryl Stearate | 2.5% |
| Caprylic/Capric Triglyceride | 10.3% |
| Avocado (Persea Gratissima) Oil | 10% |
| Cetearyl Glucoside | 1% |
| Vitamin B3 adduct | 3% |
| Glycerin | 3% |
| Water | 68% |
| Carbomer | 0.2% |
| Preservatives/pigments/perfumes/pH control agents. | 1 |

A premix "A" is prepared by mixing Stearyl Alcohol, Glyceryl Stearate, Caprylic/Capric Triglyceride, Avocado Oil, Cetearyl Glucoside and Vitamin B3 adduct. A premix "B" is prepared by mixing Glycerin and Water. Premixes "A" and "B" are separately heated at 80°C and then mixed stirring gently until cooled at about 60°C. Then the carbomer is added upon mixing. When homogenized the mixture is cooled at 40°C and the additives (Preservatives, pigments, perfumes, pH control agents) are added upon stirring. When homogeneous the mixture is cooled down.

Furthermore adducts of the present invention can be successfully used substituting the vitamin B3 compound in all compositions described in our cases US 6,528,071 and EP 1,152,742.

## Claims

1. An adduct of an amino acid or of a peptide with a vitamin B3 compound selected from niacinamide and derivatives thereof, said adduct having the following general formula:
R₁-NX-R₂
wherein
R₁ is the acylic radical of niacinamide or of a derivative thereof and R₂ is the acylic radical of an amino acid or of a peptide,
X is an organic radical or -H.

2. An adduct according to claim 1 wherein X is selected from -OH, -Alg, -CH2COOH, -CH2COOMe and -H and is preferably -H.

3. An adduct according to claim 1 or 2 wherein R₁ is the niacinamide acylic radical.

4. An adduct according to any preceding claim wherein R₂ is the acylic radical of an α-amino acid or of a peptide formed by α-amino acids.

5. An adduct according to claim 4 wherein R₂ is the acylic radical of a natural amino acid or of a peptide formed by natural amino acids.

6. An adduct according to any preceding claim for use as a medicament.

7. The use of an adduct according to any preceding claim for preparing a composition to treat vitamin B3 deficiency.

8. A cosmetic composition comprising an adduct according to claims 1 to 5.

9. A method for preparing an adduct according to claims 1 to 5 comprising the steps of:
a) providing a sterically hindered amino acid or peptide,
b) providing a vitamin B3 compound selected from niacinamide and derivatives thereof,
c) optionally activating the sterically hindered amino acid or peptide
d) forming an alkali metal salt of the vitamin B3 compound,
e) reacting the hindered amino acid and sodium salt of the vitamin B3 compound in an appropriate solvent.
